# EUROPEAN PATENT SPECIFICATION

(11) **EP 2 307 020 B1**
(45) Date of publication and mention of the grant of the patent: **20.02.2019**
(21) Application number: 09788437.3
(22) Date of filing: 29.07.2009
(51) Int. Cl.: A61K 31/4439, A61P 25/16

(54) **ADMINISTRATION REGIME FOR NITROCATECHOLS**
VERABREICHUNGSPLAN FÜR NITROCATECHOLE
RÉGIME D'ADMINISTRATION DE NITROCATHÉCHOLS

(30) Priority: 29.07.2008 US 137248 P
(43) Date of publication of application: 13.04.2011
(73) Proprietor: Bial-Portela & CA, S.A., 4745-457 S. Mamede do Coronado (PT)
(72) Inventor: DE ALMEIDA, Jose Luis, P-4540-273 Chave (PT); LEARMONTH, David Alexander, P-4445-123 Alfena (PT); ARAUJO SOARES DA SILVA, Patrício Manuel Vieira, P-4150 Porto (PT)
(74) Representative: Elkington & Fife LLP
(86) International application number: PCT/PT2009/000044
(87) International publication number: WO 2010/014025

(56) References cited:
- EP-A- 1 845 097
- WO-A-2007/013830
- WO-A-2008/094053
- LEARMONTH D A ET AL: "Chemical Synthesis and Characterization of Conjugates of a Novel Catechol-O-methyltransferase Inhibitor" BIOCONJUGATE CHEMISTRY, ACS, WASHINGTON, DC, US, vol. 13, no. 5, 29 August 2002 (2002-08-29), pages 1112-1118, XP002402315 ISSN: 1043-1802
- TERVO A J ET AL: "A STRUCTURE-ACTIVITY RELATIONSHIP STUDY OF CATECHOL-O-METHYLTRANSFER ASE INHIBITORS COMBINING MOLECULAR DOCKING AND 3D QSAR METHODS" JOURNAL OF COMPUTER-AIDED MOLECULAR DESIGN, ESCOM SCIENCE PUBLISHERS BV, XX, vol. 17, no. 12, 1 January 2003 (2003-01-01), pages 797-810, XP009037832 ISSN: 0920-654X
- "Morbus Parkinson. Stellenwert von COMT-Hemmern bestätigt // Parkinson disease. Value of COMT inhibitors is verified" MMW FORTSCHRITT DER MEDIZIN, URBAN UND VOGEL MEDIEN UND MEDIZIN VERLAGSGESELLSCHAFT, DE, vol. 143, no. 18, 3 May 2004 (2004-05-03), page 51, XP009123977 ISSN: 1438-3276

## Description

This invention relates to substituted nitrocatechols and to their use in the treatment of central and peripheral nervous system disorders according to a specified dosing regimen.

The rationale for the use of COMT inhibitors as adjuncts to L-DOPA/aromatic L-amino acid decarboxylase inhibitor (AADCi) therapy is based on their ability to reduce metabolic O-methylation of L-DOPA to 3-*O*-methyl-L-DOPA (3-OMD). The duration of L-DOPA-induced clinical improvement is brief as a result of the short *in vivo* half-life of L-DOPA which contrasts with the long half-life of 3-OMD. Additionally, 3-OMD competes with L-DOPA for transport across the blood-brain barrier (BBB), which means that only a very limited amount of an orally administered dose of L-DOPA actually reaches the site of action, i.e. the brain. Commonly, within only a few years of starting L-DOPA therapy with the usual dosage regime, L-DOPA-induced clinical improvement declines at the end of each dose cycle, giving rise to the so-called 'wearing-off' pattern of motor fluctuations. A close relationship between the 'wearing-off' phenomenon and accumulation of 3-OMD has been described (Tohgi, H., et al., Neurosci. Letters, 132:19-22, 1992). It has been speculated that this may result from impaired brain penetration of L-DOPA due to competition for the transport system across the BBB with 3-OMD (Reches, A. et al., Neurology, 32:887-888, 1982) or more simply that there is less L-DOPA available to reach the brain (Nutt, J.G., Fellman, J.H., Clin. Neuropharmacol., 7:35-49, 1984). In effect, COMT inhibition protects L-DOPA from O-methylation metabolic breakdown in the periphery, such that with repeated doses of L-DOPA, the mean plasma L-DOPA concentration is raised. In addition to reduced competition for transport into the brain, a significantly greater percentage of the orally administered dose of L-DOPA is able to reach the site of action. Thus COMT inhibition serves to increase the bioavailability of L-DOPA and the duration of antiparkinsonian action is prolonged with single doses of L-DOPA (Nutt, J.G., Lancet, 351:1221-1222, 1998).

The most potent COMT inhibitors reported thusfar are 3,4-dihydroxy-4'-methyl-5-nitrobenzophenone (Tolcapone, Australian pat. AU-B-69764/87) and (E)-2-cyano-N,N-diethyl-3-(3,4-dihydroxy-5-nitrophenyl)acrylamide (Entacapone, German pat. DE 3740383 A1).

Although sharing essentially the same pharmacophore, tolcapone differs from entacapone in that it easily enters the central nervous systems (CNS) and is able to inhibit cerebral COMT as well as peripheral COMT. Shortly after its launch, tolcapone was withdrawn from the market after several cases of hepatotoxicity were reported including three unfortunate deaths from fatal fulminant hepatitis. Today tolcapone can only be used in Parkinsonian patients who are unresponsive to other treatments and only with regular monitoring of liver function, which is expensive and inconvenient for the patient. Although the actual mechanistic causes of the liver toxicity associated with tolcapone are not fully understood, *in vitro* studies have shown that tolcapone may be reduced metabolically to reactive intermediates and it has been speculated that these may form covalent adducts with hepatic proteins resulting in hepatocellular injury (Smith, K.S. et al, Chem. Res. Toxicol., 16:123-128, 2003).

Entacapone on the other hand, although sharing the same nitrocatechol pharmacophore with tolcapone, is not associated with liver toxicity and is generally regarded as a safe drug. Unfortunately however, entacapone is a significantly less potent COMT inhibitor than tolcapone and has a much shorter *in-vivo* half-life. This means that entacapone has a very limited duration of effect and as a consequence, the drug must be administered in very high doses with every dose of L-DOPA taken by the patient. As such, the clinical efficacy of entacapone has been questioned - indeed a recent study (Parashos, S.A. et al., Clin. Neuropharmacol., 27(3): 119-123, 2004) revealed that the principal reason for discontinuation of entacapone treatment in Parkinson's disease patients was a perceived lack of efficacy.

Furthermore, the relatively short *in-vivo* half-life of known COMT inhibitors requires continuous treatment regimens normally involving the administration of several doses a day which many patients find to be burdensome. For example, tolcapone has to be administered three times a day. This factor can therefore interfere with patient compliance and quality of life.

Accordingly, there is still a need for COMT inhibitors exhibiting balanced properties of bioactivity, bioavailability and safety. In particular, there is a need for COMT inhibitors having a long *in-vivo* half-life and, thus, a prolonged action on COMT enabling fewer dosages to obtain the desired therapeutic effect.

WO 2007/013830 relates to compounds of general formula I and their use in the treatment of central and peripheral nervous system disorders.

We have now surprisingly found that, despite having a relatively short half life, compounds of general formula I are very potent COMT inhibitors endowed with exceptionally long duration of action compared to COMT inhibitors in the prior art.

Compounds of general formula I also markedly enhance the bioavailability of L-DOPA and increase the delivery of L-DOPA to the brain. The compounds significantly augment the levels of dopamine in the brain over a long period of time.

Even more surprisingly, the increased levels of L-DOPA are maintained steady over extended periods of time. These sustained effects upon both COMT activity and L-DOPA bioavailability after the administration of compounds of general formula I are markedly greater than those observed with tolcapone, the only COMT inhibitor thusfar known to be endowed with a reasonably long duration of action. (Tolcapone has a terminal half life of around 2 hours and must be administered around 3 times per day.) Furthermore, compounds of general formula I produce a steady increase in L-DOPA delivery to the brain over extended periods of time, which contrasts with that observed with tolcapone, which is prone to induce marked oscillations in the brain delivery of L-DOPA. Thus compounds of general formula I are more likely to be endowed with therapeutic advantages due to sustained constant elevation of L-DOPA levels whilst the use of tolcapone is likely to induce undesirable side-effects such as dyskinesia due to abrupt increases and decreases in L-DOPA levels.

Compounds of general formula I are compounds having the following formula 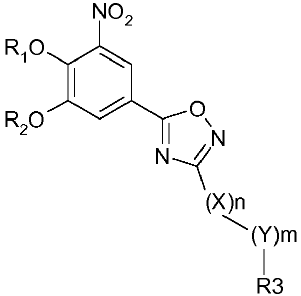 where R₁ and R₂ are the same or different and signify hydrogens, groups hydrolysable under physiological conditions, or optionally substituted alkanoyls or aroyls; X signifies a methylene group; Y represents O, S or NH; n represents 0, 1, 2 or 3; m represents 0 or 1; R₃ signifies a pyridine N-oxide group according to the formula A, B, or C, which is connected as indicated by the unmarked bond: 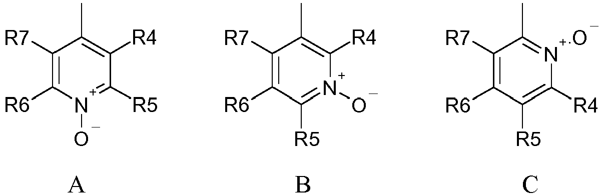 where R₄, R₅, R₆ and R₇ are the same or different, and signify hydrogen, alkyl, thioalkyl, alkoxy, aryloxy, thioaryl, alkanoyl, aroyl, aryl, amino, alkylamino, dialkylamino, cycloalkylamino, heterocycloalkylamino, alkylsulphonyl, arylsulphonyl, halogen, haloalkyl, trifluoromethyl, cyano, nitro or heteroaryl; or two or more of R₄, R₅, R₆ and R₇ taken together signify aliphatic or heteroaliphatic rings or aromatic or heteroaromatic rings; the term 'alkyl', including its variant 'alk-' in terms such as 'alkoxy', 'alkanoyl' mean carbon residues, straight or branched, containing from one to six carbon atoms; the term 'aryl' means a phenyl or naphthyl group; the term 'heterocycloalkyl' represents a four to eight-membered cyclic ring optionally incorporating at least one atom of oxygen, sulphur or nitrogen; the term 'heteroaryl' represents a five or six-membered ring incorporating at least one atom of sulphur, oxygen or nitrogen; the term 'halogen' represents fluorine, chlorine, bromine or iodine; and if R₄, R₅, R₆ and R₇ represent alkyl or aryl they are optionally substituted by one or more hydroxy, alkoxy or halogen groups; or a pharmaceutically acceptable salt or ester thereof.

Preferably, R₄, R₅, R₆ and R₇ independently from each other represent hydrogen, C₁-C₆-alkyl, C₁-C₆-thioalkyl, C₁-C₆-alkoxy, C₆-C₁₀-aryloxy, C₆-C₁₀-thioaryl, C₁-C₆-alkanoyl, C₇-C₁₁-aroyl, amino, C₁-C₆-alkylamino, di-C₁-C₆-alkylamino, C₃-C₁₂-cycloalkylamino, C₄-C₈-heterocycloalkylamino, C₁-C₆-alkylsulphonyl, C₆-C₁₀-arylsulphonyl, halogen, C₁-C₆-haloalkyl, trifluoromethyl, cyano, nitro or heteroaryl.

When R₄, R₅, R₆ and/or R₇ represent C₁-C₆-alkyl residues, preferably R₄, R₅, R₆ and/or R₇ represent methyl, ethyl, n-propyl, isopropyl, n-butyl, sec-butyl, tert-butyl, pentyl, or hexyl.

When R₄, R₅, R₆ and/or R₇ represent C₁-C₆-thioalkyl residues, preferably R₄, R₅, R₆ and/or R₇ represent thiomethyl, thioethyl, thio-n-propyl, thio-isopropyl, thio-n-butyl, thio-n-pentyl, or thio-n-hexyl.

When R₄, R₅, R₆ and/or R₇ represent C₁-C₆-alkoxy residues, preferably R₄, R₅, R₆ and/or R₇ represent methoxy, ethoxy, n-propoxy, isopropoxy, n-butoxy, sec-butoxy or tert-butoxy.

When R₄, R₅, R₆ and/or R₇ represent C₆-C₁₀-aryloxy residues, preferably R₄, R₅, R₆ and/or R₇ represent phenoxy or naphthoxy.

When R₄, R₅, R₆ and/or R₇ represent C₆-C₁₀-thioaryl residues, preferably R₄, R₅, R₆ and/or R₇ represent thiophenyl or thionaphthyl.

When R₄, R₅, R₆ and/or R₇ represent C₁-C₆-alkanoyl residues, preferably R₄, R₅, R₆ and/or R₇ represent methanoyl, ethanoyl, propanoyl or butanoyl.

When R₄, R₅, R₆ and/or R₇ represent C₇-C₁₁-aroyl residues, preferably R₄, R₅, R₆ and/or R₇ represent benzoyl or naphthoyl.

When R₄, R₅, R₆ and/or R₇ represent C₁-C₆-alkylamino residues, preferably R₄, R₅, R₆ and/or R₇ represent methylamino, ethylamino, n-propylamino, isopropylamino or n-butylamino.

When R₄, R₅, R₆ and/or R₇ represent di-C₁-C₆-alkylamino residues, preferably R₄, R₅, R₆ and/or R₇ represent dimethylamino, diethylamino, di-n-propylamino, di-n-butylamino, di-isopropylamino, methylethylamino, methylpropylamino or ethylpropylamino.

When R₄, R₅, R₆ and/or R₇ represent C₃-C₁₂-cycloalkylamino residues, preferably R₄, R₅, R₆ and/or R₇ represent pyrrolidino, piperidino, cyclohexylamino or dicyclohexylamino.

When R₄, R₅, R₆ and/or R₇ represent C₄-C₈-heterocycloalkylamino residues, preferably R₄, R₅, R₆ and/or R₇ represent morpholino, 2,6-dimethylmorpholino, 3,5-dimethylmorpholino, piperazino, N-methylpiperazino or N-ethylpiperazino.

When R₄, R₅, R₆ and/or R₇ represent C₁-C₆-alkylsulphonyl or C₆-C₁₀-arylsulphonyl residues, preferably R₄, R₅, R₆ and/or R₇ represent methylsulfonyl, ethylsulfonyl, phenylsulfonyl, or tolylsulfonyl.

When R₄, R₅, R₆ and/or R₇ represent halogen residues, preferably R₄, R₅, R₆ and/or R₇ represent chloro, bromo, iodo or fluoro.

When R₄, R₅, R₆ and/or R₇ represent C₁-C₆-haloalkyl residues, preferably R₄, R₅, R₆ and/or R₇ represent chloromethyl, fluoromethyl, dichloromethyl, difluoromethyl, trichloromethyl or trifluoromethyl.

When R₄, R₅, R₆ and/or R₇ represent heteroaryl residues, preferably R₄, R₅, R₆ and/or R₇ represent pyridyl, pyrimidyl, isoxazolyl, oxazolyl, isoxadiazolyl, oxadiazolyl, triazolyl or tetrazolyl.

When two or more of residues R₄, R₅, R₆ and R₇ taken together represent aliphatic or heteroaliphatic rings or aromatic or heteroaromatic rings, the two or more residues preferably represent aliphatic or heteroaliphatic rings or aromatic or heteroaromatic rings. Preferred combined residues are indolizinyl, isoindolyl, indolyl, indazolyl, purinyl, quinolizinyl, naphthyridinyl, isoquinolyl and quinolyl.

Where they represent aryl or alkyl, the above substituents R₄, R₅, R₆ and R₇ may optionally be substituted one or more times by hydroxy, alkoxy or halogen groups.

Details of the preparation of compounds of general formula I can be found in WO2007/013830A1.

The bioavailability, bioactivity, safety profile and other related properties known in the art (e.g. blood-brain-barrier permeability) can be routinely optimized by the skilled person on basis of the teaching of the present application by varying substituents R₁-R₇ of the above general formula I in order to obtain a desirable balanced mix of properties.

The compounds of general formula I may also be present in the form of pharmacologically acceptable salts or esters thereof. Suitable pharmaceutically acceptable counter ions are known to the art.

It is also possible to use prodrugs of compounds of the general formula I in order to alter the therapeutic profile of the active compound.

In the following description of medical indications, treatments and dosing regimens for pharmaceutical compositions containing compounds according to general formula I of the invention, the most preferred example of a compound according to the general formula I is 5-[3-(2,5-dichloro-4,6-dimethyl-1-oxy-pyridin-3-yl)-[1,2,4]oxadiazol-5-yl]-3-nitrobenzene-1,2-diol, henceforth designated as compound A, and its pharmacologically acceptable salts and esters. The half life of compound A is relatively short given its long duration of action.

Other preferred compounds of the above general formula (I) for use in the subsequent medical indications, treatments and dosing regimens include 3-(3-(3,4-dihydroxy-5-nitrophenyl)-1,2,4-oxadiazol-5-yl)-4-(trifluoromethyl)pyridine-1-oxide, 2-chloro-3-(3-(3,4-dihydroxy-5-nitrophenyl)-1,2,4-oxadiazol-5-yl)-4,6-dimethylpyridine-1-oxide, 3-(3-(3,4-dihydroxy-5-nitrophenyl)-1,2,4-oxadiazol-5-yl)-2-methyl-6-(trifluoromethyl)pyridine-1-oxide, 5-(3-(3,4-dihydroxy-5-nitrophenyl)-1,2,4-oxadiazol-5-yl)-2-(trifluoromethyl)pyridine-1-oxide, 5-(3-(3,4-dihydroxy-5-nitrophenyl)-1,2,4-oxadiazol-5-yl)-2-methyl-4-(trifluoromethyl)pyridine-1-oxide, 3-(3-(3,4-dihydroxy-5-nitrophenyl)-1,2,4-oxadiazo1-5-yl)-2,6-dimethyl-4-(trifluoromethyl)pyridine-1-oxide, 3,5-dichloro-4-(3-(3,4-dihydroxy-5-nitrophenyl)-1,2,4-oxadiazol-5-yl)pyridine-1-oxide, 3-(3-(3,4-dihydroxy-5-nitrophenyl)-1,2,4-oxadiazol-5-yl)-6-methyl-2-phenyl-4-(trifluoromethyl)pyridine-1-oxide, 2-bromo-3-(3-(3,4-dihydroxy-5-nitrophenyl)-1,2,4-oxadiazol-5-yl)-4,5,6-trimethylpyridine-1-oxide, 2-chloro-3-(3-(3,4-dihydroxy-5-nitrophenyl)-1,2,4-oxadiazol-5-yl)-4,5,6-trimethylpyridine-1-oxide, 3-(3-(3,4-dihydroxy-5-nitrophenyl)-1,2,4-oxadiazol-5-yl)-2-(trifluoromethyl)pyridine-1-oxide, 2,5-dichloro-3-(3-(3,4-dihydroxy-5-nitrophenyl)-1,2,4-oxadiazol-5-yl)-4,6-dimethylpyridine-1-oxide, 3-(3-(3,4-dihydroxy-5-nitrophenyl)-1,2,4-oxadiazol-5-yl)-5-(trifluoromethyl)pyridine-1-oxide, 3-(3-(3,4-dihydroxy-5-nitrophenyl)-1,2,4-oxadiazol-5-yl)-2-fluoropyridine-1-oxide, 4-(3-(3,4-dihydroxy-5-nitrophenyl)-1,2,4-oxadiazol-5-yl)-2-fluoropyridine-1-oxide, 2-(3-(3,4-dihydroxy-5-nitrophenyl)-1,2,4-oxadiazol-5-yl)-6-fluoropyridine-1-oxide, 2-chloro-3-(3-(3,4-dihydroxy-5-nitrophenyl)-1,2,4-oxadiazol-5-yl)-6-methylpyridine 1-oxide, 2-bromo-3-(3-(3,4-dihydroxy-5-nitrophenyl)-1,2,4-oxadiazol-5-yl)-6-methylpyridine-1-oxide, and 2-bromo-5-chloro-3-(3-(3,4-dihydroxy-5-nitrophenyl)-1,2,4-oxadiazol-5-yl)-4,6-dimethylpyridine-1-oxide and their pharmacologically acceptable salts or esters.

The present invention relates to the compounds of general formula I, their pharmaceutically acceptable salts or esters for use in the prevention or treatment of central and peripheral nervous system disorders, wherein the compound, salt or ester is administered according to a dosing regimen having a dosing periodicity ranging from about once every third day to about once weekly.

For the preparation of pharmaceutical compositions of compounds of general formula I, inert pharmaceutically acceptable carriers are admixed with the active compounds. The pharmaceutically acceptable carriers may be solid or liquid. Solid form preparations include powders, tablets, dispersible granules and capsules. A solid carrier can be one or more substances which may also act as diluent, flavouring agent, solubiliser, lubricant, suspending agent, binder, glidant, or disintegrant; it may also be an encapsulating material.

Preferably the pharmaceutical composition is in unit dosage form, e.g. a packaged preparation, the package containing discrete quantities of the preparation, for example packaged tablets, capsules and powders in vials or ampoules.

Preferably, the treated pathological states are central and peripheral nervous system-associated disorders of humans, and in particular those which benefit from administration of a COMT inhibitor. Preferably, the disorders are movement disorders including disorders involving parkinsonism, Parkinson's Disease, and restless leg syndrome. The most preferred central and peripheral nervous system associated disorder is Parkinson's Disease.

As used herein, the term treatment and variations such as 'treat' or 'treating' refer to any regime that can benefit a human or non-human animal. The treatment may be in respect of an existing condition or may be prophylactic (preventative treatment). Treatment may include curative, alleviation or prophylactic effects, such effects relating to one or more of the symptoms associated with the central and peripheral nervous system-associated disorders.

The compounds of the general formula I are used for the preparation of a medicament for the prevention or treatment of central and peripheral nervous system associated disorders according to a specified dosing regimen having a dosing periodicity ranging from about once every third day to about once weekly, i.e. administration about once every 3^{rd}, 4^{th}, 5^{th}, 6^{th} or 7^{th} day.

Suitable non-limiting starting points for dosing intervals comprise the morning, mid-day, noon, afternoon, evening, and midnight.

As used herein, the term 'effective daily dose' is the effective daily amount of compound administered when administered according to the dosing periodicity.

In the present invention, effective daily doses of compounds of general formula I are in the range of about 1 to about 900 mg/day, more preferably about 5 to about 400 mg/day, even more preferably about 25 to about 300 mg/day, even more preferably about 70 to about 200 mg/day and most preferably about 120 to about 150 mg/day.

As used herein, the term "dosage unit" refers to the amount of compound administered in each dosing periodicity.

It is preferred that individual dosage units of compounds of general formula I are in the range of about 1 to about 2400 mg, more preferably about 10 to about 1200 mg, even more preferably about 25 to about 800 mg, even more preferably about 50 to about 400 mg, and most preferably about 100 to about 200 mg.

Preferably the subject being treated with the compound of general formula I is also receiving therapy with a dopamine (DOPA) precursor and/or an AADCi.

Typical DOPA precursors include L-DOPA.

Suitable aromatic L-amino acid decarboxylase inhibitors include benserazide and carbidopa.

The compounds of general formula I, DOPA precursor and AADCi may be administered separately or in any combination. They may be administered concomitantly (for example, simultaneously) or sequentially, and with the same or differing dosing periodicity. For example, the compounds of the general formula I can be concomitantly or sequentially administered with DOPA precursor. In case of concomitant administration it is also possible to combine both or all active ingredients in one unit dosage form.

Also disclosed herein is a method of treating at least one pathological state in a patient in need thereof comprising administering about once every third day to about once weekly a pharmacologically effective dose of a compound of general formula I as defined above to the patient.

Also disclosed herein is a method for reducing COMT inhibition in a subject over 3 to 7 days, comprising administering, about once every third day to about once weekly, an effective dose of a compound of general formula I as defined above to the subject.

Also disclosed herein is a method for increasing levels of L-DOPA in the brain of a subject over 3 to 7 days, comprising administering, about once every third day to about once weekly, an effective dose of a compound of general formula I as defined above to the subject.

Also disclosed herein is a method for increasing levels of L-DOPA in the plasma of a subject over 3 to 7 days, comprising administering, about once every third day to about once weekly, an effective dose of a compound of general formula I as defined above to the subject.

Also disclosed herein is a method for decreasing levels of 3-*O-*methyl-L-DOPA (3-OMD) in the brain of a subject over 3 to 7 days, comprising administering, about once every third day to about once weekly, an effective dose of a compound of general formula I as defined above to the subject.

Also disclosed herein is a method for decreasing levels of 3-OMD in the plasma of a subject over 3 to 7 days, comprising administering, about once every third day to about once weekly, an effective dose of a compound of general formula I as defined above to the subject.

Also disclosed herein is a method for increasing bioavailability of L-DOPA in the brain of a subject over 3 to 7 days, comprising administering, about once every third day to about once weekly, an effective dose of a compound of general formula I as defined above to the subject.

Also disclosed herein is a method for increasing bioavailability of L-DOPA in the plasma of a subject over 3 to 7 days, comprising administering, about once every third day to about once weekly, an effective dose of a compound of general formula I as defined above to the subject.

The subject being treated with the compound of general formula I may also receive therapy with DOPA precursor and/or an aromatic L-amino acid decarboxylase inhibitor. Such therapy with DOPA precursor and/or AADCi may precede, follow or be simultaneous or concomitant with the treatment with the compound of general formula I.

The present invention also relates to a package comprising a pharmaceutical composition comprising 1 to 2400 mg of a compound of the general formula I in combination with instructions to administer said formulation with a dosing regimen having a dosing periodicity ranging from about once every third day to about once weekly.

### Materials and Methods

In a double-blind, randomised, placebo-controlled study aimed to investigate the tolerability, pharmacokinetics and pharmacodynamics of the compounds of general formula I, groups of eight young healthy males were administered with placebo, 5mg, 10mg, 20mg or 30mg compound A in the morning under fasting conditions for 8 days. Within each group (5mg, 10mg, 20mg or 30mg) 2 individuals were randomised to placebo and 6 to treatment.

The soluble catechol-O-methyltransferase (S-COMT) activity was expressed as the amount of metanephrine (in pmol) formed by the action of the S-COMT of washed erythrocytes, on an epinephrine substrate, per milligram of protein in the sample, per hour.

Blood samples were taken at the following times and used for preparing the washed erythrocytes:
Day 1: pre-dose, and 0.25, 0.5, 0.75, 1, 1.5, 2, 3, 4, 6, 8, 10, 12, and 16h post-dose
Days 2-7: pre-dose only
Day 8: pre-dose, and 0.25, 0.5, 0.75, 1, 1.5, 2, 3, 4, 6, 8, 10, 12, 16, 24, 36, 48, 72, 96, 120 and 144h post-dose

After centrifugation and removal of the plasma, the supernatant (uppermost erythrocyte layer) was removed and the tubes placed on ice. A volume of cold 0.9% sodium chloride solution equal to double that of the erythrocytes was then added. The erythrocytes were centrifuged and washed using this procedure three times. Centrifugation was undertaken at 4°C and at approximately 1500 g for 10 minutes. Two accurately 500 µL pipetted aliquots of washed erythrocytes were prepared and each aliquot was stored in a 2-mL tube at -70 °C until required for analysis.

Determination of S-COMT activity was carried out in compliance with Good Laboratory Practices (GLP) by HPLC with electrochemical detection.

### Description of Figures

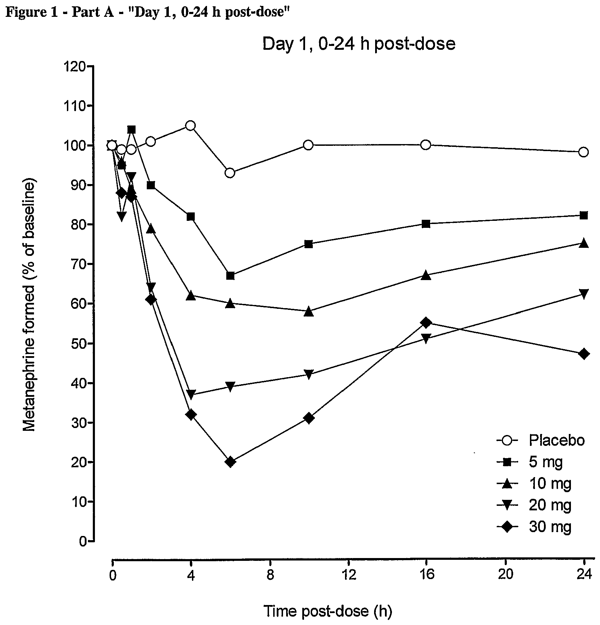

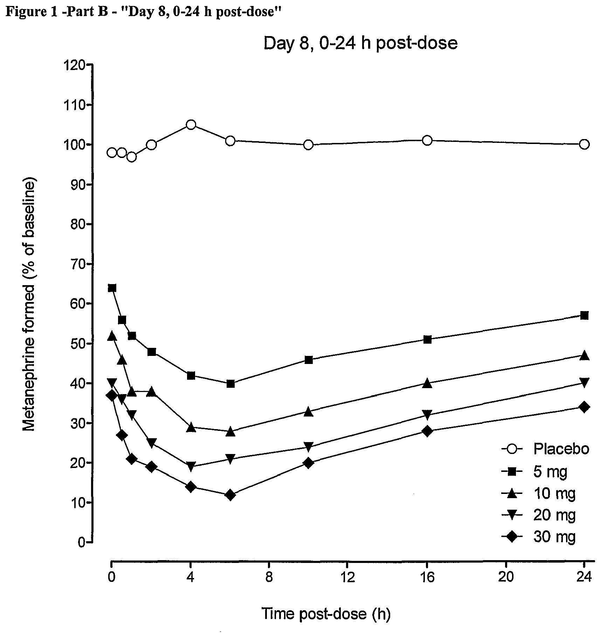

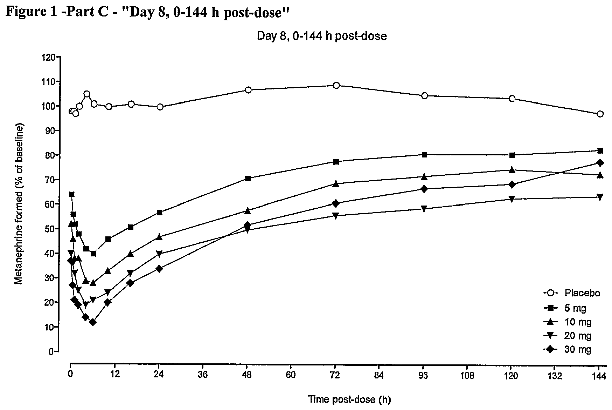

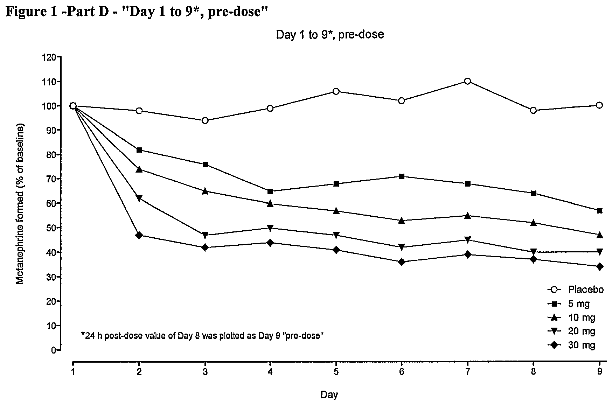

Figure 1 shows the mean S-COMT activity [expressed as the % of change of the metanephrine formed in relation to baseline (pre-dose of Day 1)] versus time profile following the first dose (Day 1) and the last dose (Day 8), and at pre-dose of an 8-day period of administration of placebo, 5mg, 10mg, 20mg or 30mg of Compound A (n=6 for each treatment group and n = 8 for each placebo group).

### Results

Figure 1 shows the mean S-COMT activity-time profile following the first dose (Figure 1A) and the last dose (Figures IB and 1C), and at pre-dose (Figure 1D) of an 8-day once daily regime with placebo, 5mg, 10mg, 20mg or 30 mg dosage of compound A (n=6 for the treatment group and n=8 for the placebo group).

Marked and sustained S-COMT inhibition was observed from the first administration and 5 days (144h) after the last dose relevant COMT inhibition was still seen, supporting the possibility of a once weekly administration regime.

The invention will now be described with reference to the following example of preparation, which is not intended to limit the invention in any way.

### Example 1 - Preparation of compound A

### (5-[3-(2,5-Dichloro-4,6-dimethyl-1-oxy-pyridin-3-yl)-[1,2,4]oxadiazol-5-yl]-3-nitrobenzene-1,2-diol)

a) To a stirred solution of 3,4-dibenzyloxy-5-nitrobenzoic acid (0.50 g, 1.319 mmol) in dimethylformamide (5 mL) at room temperature was added 1,1-carbonyldiimidazole (0.24 g, 1.45 mmol) in one portion. After stirring for ninety minutes, 2,5-dichloro-N'-hydroxy-4,6-dimethylnicotinamide (0.40 g, 1.45 mmol) was added in one portion. The resulting mixture was stirred at 135 °C for five hours and then at room temperature overnight. The reaction mixture was poured onto ice-2 N HCl (100 mL) and the resulting precipitate was filtered off, washed with water and dried in air. Recrystallisation from isopropanol gave a pale yellow solid (0.55 g, 72 %).
b) To a stirred solution of the solid obtained above (0.50 g, 0.866 mmol) in dichloromethane (20 mL) was added urea-hydrogen peroxide addition complex (0.41 g, 4.33 mmol) in one portion. The mixture was cooled in an ice-water bath and trifluoroacetic anhydride (0.73 g, 3.46 mmol) was added dropwise. The reaction mixture was allowed to stir at room temperature overnight whereupon insoluble material was filtered off. The filtrate was washed with water and brine, dried over anhydrous magnesium sulphate, filtered and evaporated. The residue was crystallised from isopropanol to give a pale yellow solid (0.35 g, 68 %).
c) To a stirred solution of the solid obtained above (0.30 g, 0.5 mmol) in dichloromethane (10 mL) at -78 °C under argon was added boron tribromide (0.38 g, 1.5 mmol) dropwise. The resulting purple suspension was allowed to stir at room temperature for one hour, then cooled again to -78 °C and carefully quenched by the addition of water. After stirring at room temperature for one hour, the precipitate was filtered off, washed with water and dried at 50 °C under vacuum to afford the desired compound as yellow crystals (0.18 g, 87 %) of m.p. 237-240 °C.

### Example 2 - Pharmaceutical formulation

Pharmaceutical formulations are prepared as follows:

**Capsule:**

| | |
|---|---|
| Compound A | 15.0% |
| Lactose monohydrate | 43.0% |
| Microcrystalline cellulose | 30.0% |
| Povidone | 4.0% |
| Croscarmellose sodium | 5.0% |
| Talc | 2.0% |
| Magnesium stearate | 1.0% |

**Capsule:**

| | |
|---|---|
| Compound A | 15.0% |
| Microcrystalline cellulose | 72.5% |
| Ethylcellulose | 5.0% |
| Sodium starch glycolate | 6.0% |
| Colloidal Silicon Dioxide | 0.5% |
| Magnesium stearate | 1.0% |

**Tablet:**

| | |
|---|---|
| Compound A | 20.0% |
| Microcrystalline cellulose | 25.0% |
| Calcium Phosphate, dibasic dihydrate | 40.0% |
| Povidone | 6.0% |
| Croscarmellose sodium | 6.0% |
| Talc | 2.0% |
| Magnesium stearate | 1.0% |

### Example 3 - Dosing regimen

Patients are treated once weekly with tablets containing 100 mg of compound of the general formula I to patients who are suffering from Parkinson's disease and who are on L-DOPA therapy. A long-lasting COMT-inhibition and a significant improvement of the clinical picture is observed.

## Claims

1. A compound of formula I 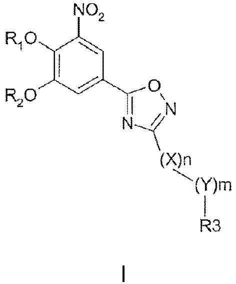 where R₁ and R₂ are the same or different and signify hydrogens, groups hydrolysable under physiological conditions, or optionally substituted alkanoyls or aroyls; X signifies a methylene group; Y represents O, S or NH; n represents 0, 1, 2 or 3; m represents 0 or 1; R₃ signifies a pyridine N-oxide group according to the formula A, B or C, which is connected as indicated by the unmarked bond: 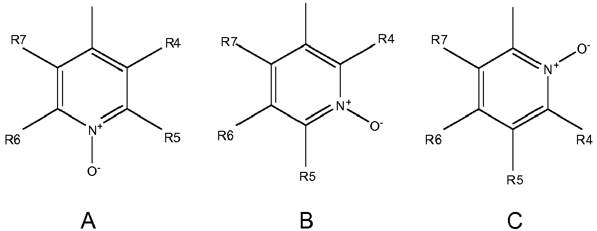 where R₄, R₅, R₆ and R₇ are the same or different, and signify hydrogen, alkyl, thioalkyl, alkoxy, aryloxy, thioaryl, alkanoyl, aroyl, aryl, amino, alkylamino, dialkylamino, cycloalkylamino, heterocycloalkylamino, alkylsulphonyl, arylsulphonyl, halogen, haloalkyl, trifluoromethyl, cyano, nitro or heteroaryl; or two or more of R₄, R₅, R₅, R₆, and R₇, taken together signify aliphatic or heteroaliphatic rings or aromatic or heteroaromatic rings; the term 'alkyl', including its variant 'alk-' in terms such as 'alkoxy' and 'alkanoyl' means carbon residues, straight or branched, containing from one to six carbon atoms; the term 'aryl' means a phenyl or naphthyl group; the term 'heterocycloalkyl' represents a four to eight-membered cyclic ring optionally incorporating at least one atom of oxygen, sulphur or nitrogen; the term 'heteroaryl' represents a five or six-membered ring incorporating at least one atom of sulphur, oxygen or nitrogen; the term 'halogen' represents fluorine, chlorine, bromine or iodine; and if R₄, R₅, R₆ and R₇ represent alkyl or aryl they are optionally substituted by one or more hydroxy, alkoxy or halogen groups; or a pharmaceutically acceptable salt or ester thereof, for use in the prevention or treatment of central and peripheral nervous system associated disorders, wherein the compound, salt or ester is administered according to a dosing regimen having a dosing periodicity ranging from about once every third day to about once weekly.

2. Compound, salt or ester for use according to claim 1, wherein the compound of formula I is 5-[3-(2,5-dichloro-4,6-dimethyl-1-oxy-pyridin-3-yl)-[1,2,4]oxadiazol-5-yl]-3-nitrobenzene-1,2-diol.

3. Compound, salt or ester for use according to claim 1 or 2, wherein the dosing regimen is once every third day, once every fourth day, once every fifth day or once every sixth day.

4. Compound, salt or ester for use according to claim 1 or 2, wherein the dosing regimen is once every seventh day.

5. Compound, salt or ester for use according to any preceding claim, wherein the starting point for a dosing interval is the morning, midday, noon, afternoon, evening or midnight.

6. Compound, salt or ester for use according to any of claims 1 to 4, wherein the starting point for a dosing interval is the evening.

7. Compound, salt or ester for use according to any of claims 1 to 5, wherein said compound, salt or ester is administered as an individual dosage unit in the range of 1 to 2400 mg.

8. Compound, salt or ester for use according to any of claims 1 to 5, wherein said compound, salt or ester is administered as an individual dosage unit in the range of 10 to 400 mg.

9. Compound, salt or ester for use according to any of claims 1 to 5, wherein said compound, salt or ester is administered as an individual dosage unit in the range of 25 to 400 mg.

10. Compound, salt or ester for use according to any preceding claim, wherein the central and peripheral nervous system associated disorder is a central and peripheral nervous system associated disorder treatable with L-DOPA/aromatic L-amino acid decarboxylase inhibitor therapy.

11. Compound, salt or ester for use according to any preceding claim, wherein the central and peripheral nervous system associated disorder is a movement disorder.

12. Compound, salt or ester for use according to claim 11, wherein the movement disorder is Parkinson's Disease.

13. Compound, salt or ester for use according to claim 11, wherein the movement disorder is restless leg syndrome.

14. Compound, salt or ester for use according to any preceding claim, wherein the compound of formula I is administered sequentially or concomitantly with L-DOPA and/or an aromatic L-amino acid decarboxylase inhibitor.

15. Compound, salt or ester for use according to claim 2, wherein the compound is used in adjunctive therapy of a subject, wherein the subject also receives therapy with L-DOPA and an aromatic L-amino acid decarboxylase inhibitor.

16. Compound, salt or ester for use according to claim 14 or 15, wherein the aromatic L-amino acid decarboxylase inhibitor is carbidopa or benserazide.

17. Package comprising a pharmaceutical composition comprising 1 to 2400 mg of a compound of formula 1, as defined in claims 1 or 2 for use as defined in claim 1, optionally further comprising L-DOPA and/or an aromatic L-amino acid decarboxylase inhibitor.

## Patentansprüche

1. Bindung der Formel I 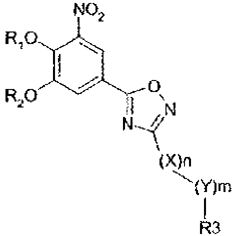 wobei R₁ und R₂ gleich oder verschieden sind und Wasserstoffe, unter physiologischen Bedingungen hydrolysierbare Gruppen oder optional substituierte Alkanoyle oder Aroyle bedeuten; X eine Methylengruppe bedeutet; Y O, S oder NH; n 0, 1, 2 oder 3 darstellt; m 0 oder 1 darstellt; R₃ eine Pyridin-N-Oxid-Gruppe gemäß der Formel A, B oder C bedeutet, die wie angegeben durch die unmarkierte Bindung verbunden ist: 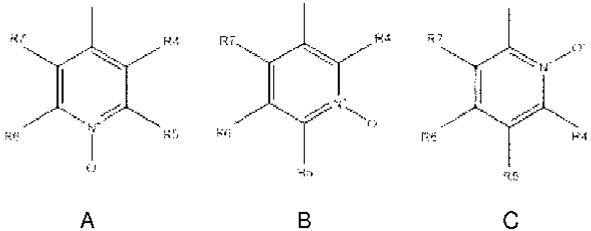 wobei R₄, R₅, R₆ und R₇ gleich oder verschieden sind und Wasserstoff, Alkyl, Thioalkyl, Alkoxy, Aryloxy, Thioaryl, Alkanoyl, Aroyl, Aryl, Amino, Alkylamino, Dialkylamino, Cycloalkylamino, Heterocycloalkylamino, Alkylsulphonyl, Arylsulphonyl, Halogen, Haloalkyl, Trifluormethyl, Cyano, Nitro oder Heteroaryl bedeuten; oder zwei oder mehr von R₄, R₅, R₅, R₆ und R₇ zusammen genommen aliphatische oder heteroaliphatische Ringe oder aromatische oder heteroaromatische Ringe bedeuten; der Begriff "Alkyl", einschließlich seine Variante "Alk-" in Begriffen wie "Alkoxy" und "Alkanoyl" Kohlenstoffreste, gerade oder verzweigt, bedeutet, die ein bis sechs Kohlenstoffatome enthalten; der Begriff "Aryl" eine Phenyl- oder Naphthylgruppe bedeutet; der Begriff "Heterocycloalkyl" einen vier- bis achtgliedrigen, zyklischen Ring darstellt, der optional mindestens ein Atom von Sauerstoff, Schwefel oder Stickstoff einschließt; der Begriff "Heteroaryl" einen fünf- oder sechsgliedrigen Ring darstellt, der mindestens ein Atom von Schwefel, Sauerstoff oder Stickstoff einschließt; der Begriff "Halogen" Fluor, Chlor, Brom oder Iod darstellt; und falls R₄, R₅, R₆ und R₇ Alkyl oder Aryl darstellen, diese optional durch eine oder mehrere Hydroxy-, Alkoxy- oder Halogengruppen substituiert sind; oder ein pharmazeutisch akzeptables Salz oder Ester davon, zur Verwendung bei der Vorbeugung oder Behandlung von mit dem zentralen und peripheren Nervensystem assoziierten Störungen, wobei die Verbindung, das Salz oder das Ester gemäß einem Dosierungsregime mit einer Dosierungsperiodizität im Bereich von etwa einmal jeden dritten Tag bis etwa einmal wöchentlich verabreicht wird.

2. Verbindung, Salz oder Ester zur Verwendung gemäß Anspruch 1, wobei die Verbindung der Formel I 5-[3-(2,5-Dichlor-4,6-dimethyl-1-oxy-pyridin-3-yl)-[1,2,4]oxadiazol-5-yl]-3-nitrobenzol-1,2-diol ist.

3. Verbindung, Salz oder Ester zur Verwendung gemäß Anspruch 1 oder 2, wobei das Dosierungsregime einmal jeden dritten Tag, einmal jeden vierten Tag, einmal jeden fünften Tag oder einmal jeden sechsten Tag ist.

4. Verbindung, Salz oder Ester zur Verwendung gemäß Anspruch 1 oder 2, wobei das Dosierungsregime einmal jeden siebten Tag ist.

5. Verbindung, Salz oder Ester zur Verwendung gemäß einem vorhergehenden Anspruch, wobei der Anfangspunkt für ein Dosierungsintervall der Morgen, der Mittag, die Mittagstunde, der Nachmittag, der Abend oder Mitternacht ist.

6. Verbindung, Salz oder Ester zur Verwendung gemäß einem der vorhergehenden Ansprüche 1 bis 4, wobei der Ausgangspunkt für ein Dosierungsintervall der Abend ist.

7. Verbindung, Salz oder Ester zur Verwendung gemäß einem der vorhergehenden Ansprüche 1 bis 5, wobei die Verbindung, das Salz oder das Ester als individuelle Dosierungseinheit im Bereich von 1 bis 2400 mg verabreicht wird.

8. Verbindung, Salz oder Ester zur Verwendung gemäß einem der vorhergehenden Ansprüche 1 bis 5, wobei die Verbindung, das Salz oder das Ester als individuelle Dosierungseinheit im Bereich von 10 bis 400 mg verabreicht wird.

9. Verbindung, Salz oder Ester zur Verwendung gemäß einem der vorhergehenden Ansprüche 1 bis 5, wobei die Verbindung, das Salz oder das Ester als individuelle Dosierungseinheit im Bereich von 25 bis 400 mg verabreicht wird.

10. Verbindung, Salz oder Ester zur Verwendung gemäß einem vorhergehenden Anspruch, wobei die mit dem zentralen und peripheren Nervensystem assoziierte Störung eine mit dem zentralen und peripheren Nervensystem assoziierte Störung ist, die mit L-DOPA/aromatischer L-Aminosäure-Decarboxylase-Inhibitor-Therapie behandelbar ist.

11. Verbindung, Salz oder Ester zur Verwendung gemäß einem vorhergehenden Anspruch, wobei die mit dem zentralen und peripheren Nervensystem assoziierte Störung eine Bewegungsstörung ist.

12. Verbindung, Salz oder Ester zur Verwendung gemäß Anspruch 11, wobei die Bewegungsstörung Parkinson-Erkrankung ist.

13. Verbindung, Salz oder Ester zur Verwendung gemäß Anspruch 11, wobei die Bewegungsstörung Restless-Leg-Syndrom ist.

14. Verbindung, Salz oder Ester zur Verwendung gemäß einem vorhergehenden Anspruch, wobei die Verbindung der Formel I sequenziell oder gleichzeitig mit L-DOPA und/oder einem aromatischen L-Aminosäure-Decarboxylase-Inhibitor verabreicht wird.

15. Verbindung, Salz oder Ester zur Verwendung gemäß Anspruch 2, wobei die Verwendung bei einer Begleittherapie eines Subjekts verwendet wird, wobei das Subjekt auch eine Therapie mit L-DOPA und einem aromatischen L-Aminosäure-Decarboxylase-Inhibitor erhält.

16. Verbindung, Salz oder Ester zur Verwendung gemäß Anspruch 14 oder 15, wobei der aromatische L-Aminosäure-Decarboxylase-Inhibitor Carbidopa oder Benserazid ist.

17. Verpackung, die eine pharmazeutische Zusammensetzung beinhaltet, die 1 bis 2400 mg einer Verbindung der Formel 1 beinhaltet, wie definiert in den Ansprüchen 1 oder 2, zur Verwendung, wie definiert in Anspruch 1, die optional ferner L-DOPA und/oder einen aromatischen L-Aminosäure-Decarboxylase-Inhibitor beinhaltet.

## Revendications

1. Composé de formule I 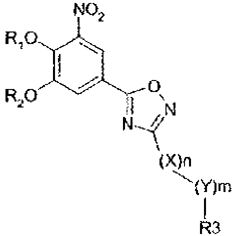 où R₁ et R₂ sont identiques ou différents et signifient des hydrogènes, des groupes hydrolysables dans des conditions physiologiques, ou des alcanoyles ou aroyles facultativement substitués ; X signifie un groupe méthylène ; Y représente O, S ou NH ; n représente 0, 1, 2 ou 3 ; m représente 0 ou 1 ; R₃ signifie un groupe pyridine N-oxyde selon la formule A, B ou C, qui est connecté comme est indiqué par la liaison non marquée : 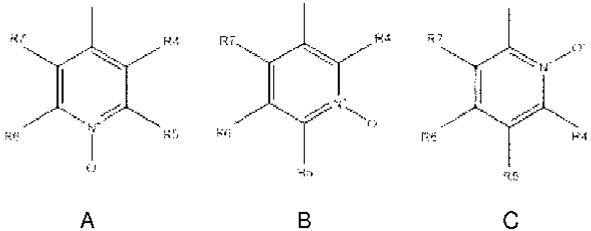 où R₄, R₅, R₅ sont R₇ sont identiques ou différents et signifient un hydrogène, un alkyle, un thioalkyle, un alcoxy, un aryloxy, un thioaryle, un alcanoyle, un aroyle, un aryle, un amino, un alkylamino, un dialkylamino, un cycloalkylamino, un hétérocycloalkylamino, un alkylsulfonyle, un arylsulfonyle, un halogène, un haloalkyle, un trifluorométhyle, un cyano, un nitro ou un hétéroaryle ; ou deux de R₄, R₅, R₅, R₆, et R₇, ou plus, pris ensemble signifient des noyaux aliphatiques ou hétéroaliphatiques ou des noyaux aromatiques ou hétéroaromatiques ; le terme « alkyle », incluant sa variante « alc- » dans des termes tels que « alcoxy » et « alcanoyle », signifie des résidus carbonés, linéaires ou ramifiés, contenant un à six atomes de carbone ; le terme « aryle » signifie un groupe phényle ou naphtyle ; le terme « hétérocycloalkyle » représente un noyau cyclique de quatre à huit éléments contenant facultativement au moins un atome d'oxygène, de soufre ou d'azote ; le terme « hétéroaryle » représente un noyau de cinq ou six éléments contenant au moins un atome de soufre, d'oxygène ou d'azote ; le terme « halogène » représente le fluor, le chlore, le brome ou l'iode ; et si R₄, R₅, R₅ et R₇ représentent l'alkyle ou l'aryle, il sont facultativement substitués par un ou plusieurs groupes hydroxy, alcoxy ou halogène ; ou un sel ou un ester pharmaceutiquement acceptable de celui-ci, destiné à être utilisé dans la prévention ou le traitement de troubles associés aux systèmes nerveux central et périphérique, dans lequel le composé, le sel ou l'ester est administré selon un schéma posologique ayant une périodicité d'administration allant d'environ un tous les trois jours à environ une fois par semaine.

2. Composé, sel ou ester destiné à être utilisé selon la revendication 1, dans lequel le composé de formule I est le 5-[3-(2,5-dichloro-4,6-diméthyl-1-oxy-pyridin-3-yl)-[1,2,4] oxadiazol-5-yl]-3-nitrobenzène-1,2-diol.

3. Composé, sel ou ester destiné à être utilisé selon la revendication 1 ou 2, dans lequel le schéma posologique est une fois tous les trois jours, une fois tous les quatre jours, une fois tous les cinq jours ou une fois tous les six jours.

4. Composé, sel ou ester destiné à être utilisé selon la revendication 1 ou 2, dans lequel le schéma posologique est une fois tous les sept jours.

5. Composé, sel ou ester destiné à être utilisé selon l'une quelconque des revendications précédentes, dans lequel le point de départ pour un intervalle d'administration est le matin, la mi-journée, le midi, l'après-midi, le soir ou minuit.

6. Composé, sel ou ester destiné à être utilisé selon l'une quelconque des revendications 1 à 4, dans lequel le point de départ pour un intervalle d'administration est le soir.

7. Composé, sel ou ester destiné à être utilisé selon l'une quelconque des revendications 1 à 5, dans lequel ledit composé, sel ou ester est administré sous forme d'une dose unitaire individuelle dans la plage de 1 à 2 400 mg.

8. Composé, sel ou ester destiné à être utilisé selon l'une quelconque des revendications 1 à 5, dans lequel ledit composé, sel ou ester est administré sous forme d'une dose unitaire individuelle dans la plage de 10 à 400 mg.

9. Composé, sel ou ester destiné à être utilisé selon l'une quelconque des revendications 1 à 5, dans lequel ledit composé, sel ou ester est administré sous forme d'une dose unitaire individuelle dans la plage de 25 à 400 mg.

10. Composé, sel ou ester destiné à être utilisé selon l'une quelconque des revendications précédentes, dans lequel le trouble associé aux systèmes nerveux central et périphérique est un trouble associé aux systèmes nerveux central et périphérique pouvant être traité avec une thérapie à base de L-DOPA/d'inhibiteur de la décarboxylase des acides L-aminés aromatiques.

11. Composé, sel ou ester destiné à être utilisé selon l'une quelconque des revendications précédentes, dans lequel le trouble associé aux systèmes nerveux central et périphérique est un trouble du mouvement.

12. Composé, sel ou ester destiné à être utilisé selon la revendication 11, dans lequel le trouble du mouvement est la maladie de Parkinson.

13. Composé, sel ou ester destiné à être utilisé selon la revendication 11, dans lequel le trouble du mouvement est le syndrome des jambes sans repos.

14. Composé, sel ou ester destiné à être utilisé selon l'une quelconque des revendications précédentes, dans lequel le composé de formule I est administré de manière séquentielle ou concomitante avec de la L-DOPA et/ou un inhibiteur de la décarboxylase des acides L-aminés aromatiques.

15. Composé, sel ou ester destiné à être utilisé selon la revendication 2, dans lequel le composé est utilisé dans une thérapie d'appoint d'un sujet, dans lequel le sujet reçoit aussi une thérapie à base de L-DOPA et d'un inhibiteur de la décarboxylase des acides L-aminés aromatiques.

16. Composé, sel ou ester destiné à être utilisé selon la revendication 14 ou 15, dans lequel l'inhibiteur de la décarboxylase des acides L-aminés aromatiques est la carbidopa ou le bensérazide.

17. Paquet comprenant une composition pharmaceutique comprenant 1 à 2 400 mg d'un composé de formule 1, comme est défini dans les revendications 1 ou 2 destiné à être utilisé selon la revendication 1, comprenant en outre facultativement de la L-DOPA et/ou un inhibiteur de la décarboxylase des acides L-aminés aromatiques.
